# EUROPEAN PATENT SPECIFICATION

(11) **EP 3 704 292 B1**
(45) Date of publication and mention of the grant of the patent: **22.03.2023**
(21) Application number: 18801051.6
(22) Date of filing: 02.11.2018
(51) Int. Cl.: D04H 1/425, D04H 1/435, D04H 1/498, A61F 13/511

(54) **ABSORBENT ARTICLE COMPONENT**
ABSORBIERENDER ARTIKELBESTANDTEIL
COMPOSANT D'ARTICLE ABSORBANT

(30) Priority: 03.11.2017 DK PA201770824
(43) Date of publication of application: 09.09.2020
(73) Proprietor: Jacob Holm & Sons AG, 4052 Basel (CH)
(72) Inventor: PETERSEN, Katharine Dyrmose, 4052 Basel (CH); KNOWLSON, Richard, 4052 Basel (CH)
(74) Representative: Chas. Hude A/S
(86) International application number: PCT/IB2018/058609
(87) International publication number: WO 2019/087138

(56) References cited:
- EP-A1- 1 614 790
- EP-A1- 2 305 749
- EP-A2- 1 748 100
- WO-A1-2007/114742
- US-A1- 2008 003 908

## Description

### FIELD OF THE INVENTION

The present invention relates to a core cover for the absorbent core in absorbent products.

### BACKGROUND OF THE INVENTION

Absorbent products such as diapers need to hold liquid and in order to do so, the core of such absorbent products typically comprises a mix of cellulosic fluff pulp and super absorbent polymers (SAP). Nowadays, increasing amounts of the SAP is used to reduce weight and particularly thickness. One aspect of using more SAP can be gel blocking, whereby some of the SAP is wet and swells, thereby blocking the path for more liquid to get to the remaining (non-saturated) SAP thus making the product less efficient and comfortable as the absorbent core may feel lumpy due to differential swelling. Further, SAP requires a certain amount of time to absorb expelled liquid, which may not be given when used in diapers.

Typically, the production of such products comprising an absorbent core involves prepreparing the pulp core as an airlaid sheet (or by other formation methods) and where said sheet is rolled out along with the other component layers making up the absorbent product, e.g. the diaper. However, such a sheet may feel stiff and does not provide optimum movement and comfort when the product is worn. Having such a sheet also restricts how the SAP can be added as this is typically provided as granules.

Another method involves using loose fluff with which the SAP granules are mixed prior to assembly in the absorbent product. However, this loose fluff and/or SAP needs to be controlled and contained which leads to the development of core covers/wraps or "bags" for the core material (mix of loose fluff and SAP).

Typically, core covers are made of lightweight spunbond/meltblown fibres or tissue. In both of these cases, the material may be considered lightweight (or very lightweight) of the order of 8-10 gsm (grams per square meter) up to around 20 gsm.

The spunbond/meltblown core cover has the advantages of having a relatively high strength at a low weight and having the potential to be heat or sonic welded into the absorbent product. This core cover is not absorbent in itself and may thus be considered passive in the absorbent sense and may thus not provide any further functionality to the product. This passiveness may or may not be addressed by special finishing in order to adjust the hydrophilic or hydrophobic properties to allow liquid to penetrate into the absorbent core which is the aim of the core cover.

Tissue is a second option for making a core cover which is also considered lightweight and may be more active in regard to absorbency. However, tissue may be considered too absorbent. Further, lightweight tissue is considered relatively weak and is thus likely to break/tear during high-speed manufacturing processes. Further, the wet integrity of the tissue is likewise considered relatively weak.

Finally, very lightweight and thin materials provide less protection or cushioning effect in the end product, e.g. the diaper, and thus, granules of the dry SAP may be felt at the surface or could even puncture the core cover.

Various absorbent products are known in the art, but none of them with a functional core. EP 1614790A1 describes a non-wowen functional core, but here is no disclosure of a non-wowen material attached to a functional core. constitutes the core itself. WO 2007/114742 A1 describes non-wowen fabrics, but not in combination with any kind of functional cores. Similarly, EP 2305749 A1 only describes absorbing fabrics but not in combination with any kind of core. EP 1748 100 A2 describes a non-wowen structure to be applied as a top sheet of a napkin, but again there is no functional cores. Also, in US2008/003908 A1 there is description of absorbing layers, but without a functional core.

### GENERAL DESCRIPTION

It is an object of the invention to solve some of the above-mentioned problems. This is achieved by a non-woven functional core cover for use in combination with an absorbent core, where said functional core cover comprises at least a first fraction comprising lyocell fibres, and characterised in that a majority of the fibres constituting the functional core cover are oriented in the same direction. Thus, the functional core cover may be considered an absorbent article component. Further, it is noted that other fractions may be included, since it is a majority of the fibres constituting the functional core cover as a whole which should be oriented in the same direction, and not necessarily the lyocell fibres only.

By a non-woven product should be understood a type of fabric based on textile fibres being physically bonded or entangled. The skilled person should understand that multiple bonding techniques exist, but hydroentanglement, also known as spunlacing, is a preferred technique in the present invention.

By a core cover should be understood a type of material or fabric used to wrap, encapsulate, cover, or in any other way contain a core. Preferably, the core is an absorbent core comprising absorbent materials such as cellulosic fluff and/or super absorbent polymers (SAP). The core cover is considered functional due to its properties.

By a majority should be understood, that at least 50 % of the fibres are oriented in the same direction. This may also be expressed in terms of the tensile strength of the fabric, where the tensile strength should be greater in the direction in which a majority of the fibres are oriented, compared to any other direction, e.g. a direction perpendicular to the direction in which a majority of the fibres are oriented.

Lyocell in the present case should be understood as a sub-category of rayon. More specifically, the lyocell is a man-made cellulosic material, which is composed of cellulose precipitated from an organic solution in which no substitution of the hydroxyl groups takes place and no chemical intermediates are formed. This definition reflects the definition as used by the US Federal Trade Commission. During the manufacturing process of the lyocell fibres, the individual chains of cellulose molecules are primarily oriented in the same direction being in the longitudinal extension of the fibre, thereby giving the fibres a "nano orientation" beneficial for the purpose of the present invention.

Thereby, a functional core cover having improved wicking properties and improved wet integrity is realised. Such advantages are especially relevant when used in combination with an absorbent core, for example in a diaper. However, the person skilled in the art would understand that the desire for a fabric having improved wicking properties is present in multiple industries since such wicking properties allow a distribution of a liquid in contact with the fabric. Further, the wet integrity is relevant to consider whenever a fabric becomes wet since it is known that the presence of a liquid may compromise the functionality of said fabric.

For the remaining part of this document, the functional core cover is described in relation to the absorbent core in a diaper, but the skilled person acknowledges that the functional core cover may be used in similar end products, including sanitary towels, absorbent bandages, etc. Common for these products is the ability to absorb and hold a liquid.

When used to encapsulate an absorbent core, e.g. in a diaper, the advantages of the functional core cover include allowing reducing the amount of fluff used in the mix with SAP and improved wicking properties. A reduction of material used in the absorbent core improves the feeling of wearing the absorbent product, e.g. a baby diaper, which in turn facilitates the movement of the baby. Further, a reduction of fluff allows making the absorbent core thinner, which in turn reduces the risk of leakages since thick absorbent cores are less flexible.

Improved wicking properties are ensured through the use of the material as disclosed, the use of a non-woven product, and the orientation of the fibres. The wicking may be measured according to the DIN 53924 standard. The wicking properties cause liquid to be distributed better across the surface of the core cover, such that more of the absorbent core is exposed to the expelled liquid. Enhanced wicking properties are especially relevant where a large amount of liquid is expelled locally. In such case, absorbent cores covered by core covers known from prior art would have difficulties absorbing the large amount of liquid since the SAP requires a certain amount of time to initiate absorption and to absorb all the liquid. Thus, the liquid not absorbed is readily at risk of leaking or introducing an unpleasant feeling to the wearer due to the liquid being in contact with the skin. Moreover, due to the weak wicking properties of core covers known from prior art, some SAP and/or fluff in the absorbent core may remain dry despite other parts being saturated. Due to the wicking properties of the functional core cover according to the invention, the locally expelled liquid is more easily carried away from the point of depletion. This ensures that more SAP absorbs liquid, and thus reduces the risk of excess liquid causing a leak or unpleasant feeling. Further, the properties of the functional core cover ensure that part of the liquid may be absorbed by the core cover before being carried into the absorbent core, which takes away the instant "pressure" on the SAP in the event of liquid expelling thereby allowing the SAP to react, i.e. initiate absorption.

The orientation of the fibres according to the invention ensures that the direction of the liquid distribution may be controlled. When used in a diaper product, the functional core cover is commonly shaped into a rectangle due to the dimensioning of the human body. When shaped into such a rectangle, it is desired to have the liquid distributed in its length since this carries the liquid further distances away. To control this distribution, the functional core cover according to the invention is especially advantageous since the orientation of the fibres within said functional core cover allows controlling the direction of the wicking. More specifically, when a majority of the fibres of the functional core cover according to the invention are oriented in the same direction, wicking is experienced primarily in said same direction. This is true, since liquid is more easily wicked along the fibre surface, but may as well be wicked through the fibre when the liquid is absorbed by the fibre. Wicking within the fibres is enhanced by the fact that the individual cellulose chains may be lined up or "nano-oriented" in the length of the fibre as described briefly above. Such internal wicking is especially experienced in lyocell fibres due to their inherent properties and manufacturing technique. On the contrary, liquid needs to overcome a larger energy barrier when jumping between fibres thus limiting the tendency to wick across fibres. This is founded in elementary surface physics and the theory of surface tension and energy.

The "same direction" should be understood such that the fibres are oriented primarily in the same direction, but the skilled person acknowledges that due to the flexible nature of fibres, this is not always the case. As a way to characterise the orientation of the fibres, the tensile strength of the fabric may be considered as is the case in the industry. More specifically, the functional core cover according to the invention should have a higher tensile strength in the direction in which a majority of the fibres are oriented than in the cross direction, i.e. the direction perpendicular to this direction. The direction in which a majority of the fibres are oriented may be known as the machine direction.

A further advantage of the functional core cover is the increased wet integrity over prior art. By wet integrity should be understood the resistance to tear and/or break when wet. A wet state may be present when used in combination with an absorbent core. Thus, an increased wet integrity implies a reduced risk of tear when wet.

An even further advantage of the functional core covers as disclosed is the avoidance of the use of plastic-based polymers such as polypropylene (PP) and polyester (PES/PET). Instead, lyocell is a product based on wood pulp which reduces the environmental impact of the product. Means for reducing the environmental impact is especially relevant to consider in respect to products intended for one-time use only, such as most diapers today. Thus, the avoidance of use of PP and/or PET in a majority of the functional core cover qualifies the product to e.g. the EU Ecolabel.

In an embodiment, the functional core cover may comprise a second fraction comprising viscose rayon fibres.

The core cover may be made up of a plurality of fractions of textile fibres. In such an embodiment, the first fraction of lyocell fibres may constitute a percentage P of the functional core cover, and a second fraction of viscose rayon fibres may constitute a corresponding percentage (100 % - P) of the functional core cover. In further embodiments, further fractions may be added, with or without affecting the ratio between lyocell and viscose rayon.

In the present application, viscose rayon should be understood as a term for cellulose xanthate which is rayon made by using a specific viscose process. In said viscose process, wood pulp is treated with carbon disulfide, whereby the cellulose in the wood pulp is converted into cellulose xanthate. Thus, another name for viscose rayon is xanthate rayon. Said process is considered the standard process for making viscose rayon and is thus not limiting to the purpose of the present invention in case other processes of making viscose rayon become available. The skilled person notes that lyocell and viscose rayon are two sub-categories of rayon. Although in certain cases, "viscose" is considered a synonym for "rayon", the present use of the word "viscose" is used for simplicity instead of "viscose rayon".

The use of viscose allows manipulating the bulkiness and softness of the functional core cover without affecting its wicking and strength properties. The viscose may also be included due to cost optimisations. In a preferred embodiment, the functional core cover comprises about 30 wt% lyocell fibres and about 70 wt% viscose fibres. However, the amount of viscose may vary from 0 wt% to 95 wt%, but an amount of viscose fibres between 50 wt% and 80 wt% is preferred, or even more preferred is the range between 65 wt% and 75 wt%. In the even more preferred range of viscose, the corresponding amount of lyocell fibres is 25 wt% to 35 wt%.

In an embodiment, the absorbent core may comprise at least a super absorbent polymer (SAP).

SAP may be characterised by its ability to absorb and retain large or extremely large amounts of liquid relative to its own mass. The absorbency of the SAP may be between 50 times its own weight and up to 300 times its own weight. However, the absorbency depends on the composition of the liquid and therefore, absorbencies below 50 times its own weight are also expected within the scope of the invention.

A preferred type of SAP may be made from the polymerisation of acrylic acid blended with sodium hydroxide in the presence of an initiator to form a poly-acrylic acid sodium salt (sodium polyacrylate) which is the most common type of SAP made today. Other types of SAP include polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile.

Due to the super absorbent properties, prolonged contact with the skin should be avoided since such contact may desiccate the skin. Thus, it is an aspect of the invention to provide a core cover having a sufficiently wet integrity such that the risk of tearing or breakage of said core cover during use in the end product is reduced.

The SAP may be provided as granules, whereby the SAP achieves a large surface area and further allows an even distribution in the entire absorbent core. The granules may have a size below 4 mm, or below 1 mm.

In a functional core cover according to the invention, the provided cushioning effect reduces the feeling of the presence of granules in the absorbent core. Further, the provided strength ((wet) integrity) reduces the risk of the granules penetrating or tearing the functional core. In other words, the functional core cover reduces the risk of SAP granules escaping the absorbent core, which would cause skin irritation in case of skin contact.

In an embodiment, the absorbent core may further comprise fluff pulp. Fluff pulp is a type of chemical pulp made from long fibre softwoods. Fluff pulp may also be referred to as cellulosic fluff pulp or cellulosic fluff. The fluff pulp is used due to its water absorbency and bulk volume. The fluff pulp cooperates with the SAP, such that said fluff pulp may absorb the instantly expelled liquid thereby giving the SAP some time to initiate the absorption of the liquid in a subsequent step. Thus, fluff pulp is necessary to take away the instant "pressure" on the SAP when liquid is expelled onto the absorbent core. Further, fluff is used as a carrier for the SAP thereby ensuring that the SAP is distributed evenly in the absorbent core.

When covering the absorbent core with a functional core cover according to the invention, the amount of fluff may be reduced since the absorbency and wicking properties of the core cover may absorb some of the liquid before it reaches the fluff pulp. This absorbed liquid may then be wicked/distributed towards dry portions of the SAP, thereby ensuring that more SAP is utilised in the absorbent core which is especially relevant in case of a large liquid expelling. This opposes prior art, where the core cover does not provide means, i.e. appropriately chosen materials and accompanying fibre orientation, for wicking and carrying away the liquid from the point of depletion. Thus, prior art results in dry SAP in some parts of the absorbent core, while other parts are completely saturated, which increases the risk of leakage since the liquid cannot be distributed.

In an embodiment, the non-woven core cover may be made in a hydroentanglement process.

Hydroentanglement is also known as spunlacing. Hydroentanglement is a fabrication technique wherein the individual fibres are entangled using high-pressure water jets. When fabricating a non-woven fabric using hydroentanglement, a plurality of non-entangled individual fibres is arranged in a fibrous web using at least one carding machine followed by moving said fibrous web into a hydroentanglement section, wherein multiple high-pressure water jets are introduced, said water jets penetrating the fibrous web and causing entanglement and thereby (mutual) physical bonding of the textile fibres. Thereby, the entanglement of textile fibres creates a non-woven fabric. The fibrous web may be formed using at least one carding machine, but may also be formed by other means.

The movement of the fibrous web between the carding machine and the hydroentanglement section may be carried out by means of a conveyor belt, the speed of said movement partly affecting the orientation of the fibres. In other words, the fibrous web is laid down on a conveyor belt by the carding machine and subsequently transported into the hydroentanglement section.

Preferably and additionally, the carding machine forming the fibrous web serves to arrange a majority of the fibres in the same direction, such that both said carding machine, the movement of the conveyor belt, and the hydroentanglement section cooperate to arrange a majority of the fibres in the same direction according to the invention. A second aspect responsible for orienting a majority of the fibres in the same direction is the combination of the fibre densities, the preferred fibre lengths, and the action of the carding machine to the fibres. In other words, the disclosed ranges of density and length partly ensure that a majority of the fibres become oriented - but it also depends on settings of the machinery, i.e. the carding machine and the hydroentanglement section.

Hydroentanglement is commonly employed to non-woven fabric having a relatively low weight due to the limitations set by the use of water jets. A relatively low weight may be less than 100 gsm (grams per square meter, g/m²). The direction in which the fibrous web flows into the hydroentanglement section is known as the machine direction.

Thereby, a majority of the fibres used in the functional core cover according to the invention become oriented in the same direction which enhances its wicking and strength properties. Further, the look and feel from hydroentanglement are achieved. Further, hydroentanglement provides the possibility of making a fabric, including the functional core cover, lightweight.

In an embodiment, the majority of the fibres of the functional core cover may be oriented in the machine direction of the functional core cover, the machine direction being the direction in which a precursor to the functional core cover flows into a hydroentanglement section for bonding in the hydroentanglement process.

The precursor may be the fibrous web as described previously. By flowing into a hydroentanglement section is meant that e.g. a conveyor belt transports/moves the fibrous web into said hydroentanglement section. The hydroentanglement section should be understood as the machinery and part of the process, wherein the actual bonding of the fibres within the fibrous web takes place. The orientation of the fibres may be controlled by both the carding machine laying down the fibrous web, the speed and movement of the conveyor belt, and the hydroentanglement section. In addition to these settings of the machinery, the orientation of the fibres is to a great extent dependent on the density of the fibres, the length of the fibres, and the weight of the fabric when assembled.

In an embodiment, the functional core cover may be flat, patterned, or apertured.

Such surface shaping may be introduced in the hydroentanglement process or in a subsequent process, e.g. using a press. The shaping may be considered as contours in the otherwise planar/flat fabric/core cover. Shaping such as patterns and apertures may manipulate the wicking properties, such that the inherent wicking properties of the functional core cover according to the invention may be enhanced. However, the apertures should be of a size smaller than the minimum size of the SAP granules, such that said granules do not escape the absorbent core covered by the functional core cover.

In an embodiment, the functional core cover may further comprise a third fraction comprising polypropylene fibres and/or polyester fibres.

Polypropylene (PP) fibres and/or polyester (PES) fibres may be included for multiple reasons, including cost considerations, and the possibility of sonic bonding the core cover due to the presence of plastic-based polymers. A preferred type of polyester (PES) is polyethylene terephthalate (PET). The PP and/or PET may be included as a blend in a third fraction, or individually as a third and a fourth fraction. For example, when both lyocell and viscose is used, the ratio between the first and second fractions may or may not be constant despite adding a third and/or a fourth fraction of PP and/or PET. Thus, adding PP and/or PET to the functional core cover according to the invention inevitably affects the amount of lyocell and viscose in the core cover, but the mutual ratio between lyocell and viscose may be unaffected. Likewise, the addition of PP and/or PET may be added on the expense of viscose only, such that the amount by weight or percentage of lyocell is the same, even before the addition of PP and/or PET. For example, a fibrous web comprising 30 % lyocell and 70 % viscose may be added to an amount of PP and/or PET, but without affecting the percentage of lyocell. Thus, a new composition could be 30 % lyocell, 50 % viscose, and 20 % PP and/or PET. The same applies in the opposite case where the amount of viscose is kept constant.

For example, a maximum of 50 % of the functional core cover may comprise PP and/or PET.

In an embodiment, the basis weight of the functional core cover may be between 15 gsm and 30 gsm, or more specifically between 18 gsm and 22 gsm.

Thereby, the functional core cover may be considered lightweight which is a desired property in the foreseen uses. Further, the weights are achievable using hydroentanglement, whereby the feel of a hydroentangled non-woven fabric is transferred to the feel of the functional core cover.

Having a lightweight fabric is desirable in the present invention for multiple reasons. Firstly, a low weight increases the comfort of the product when worn due to the reduced thickness of the product and further reduces the cost of the product. Secondly, the lower weight inevitably reduces the amount of material when comparing to a like product being heavier. The reduced amount of material allows the functional core cover to act as a transportation/distribution layer rather than an absorption layer which would be expected if the amount of material were higher. In other words, a too heavy fabric would retain a large amount of liquid, meaning less liquid is transported into the absorbent core and/or distributed across the surface of said absorbent core for subsequent absorption, the latter being an objective of the present invention. Thus, a lightweight fabric having a basis weight between 15 gsm and 30 gsm, or more specifically 18 gsm to 22 gsm, or even more specifically 20 gsm, is desired for use in a functional core cover according to the invention.

In an embodiment, the density of the fibres may be between 1 dtex and 3.3 dtex.

Thereby, the low density of the fibres adds to the lightweight properties of the fabric. Further, the low density of the fibres allows the basis weight of the fabric to be reduced in case a fixed number of fibres is needed to form a fabric having the desired strength, opacity, softness, and volume. By density is meant the yarn count expressed in terms of mass per unit length. In the present case, the unit used is dtex, which is equal to the number of grams per 10,000 m. The use of finer (< 1 dtex) fibres or coarser (> 3.3 dtex) fibres are foreseen within the present invention.

Further, the fibres may comprise varying cross sections. For example, the cross section of the fibres may be round, flat, trilobal, multilobal, triangular, hollow, solid, etc. The use of certain cross sections may be particularly relevant for enhancing the wicking properties along the surface and/or within the fibre itself. For example, a hollow fibre may more easily wick liquid in its interior due to capillary forces, and a fibre having a large surface area, such as a trilobal fibre, may more easily wick liquid on its outer surface due to its surface area.

In an embodiment, the fibre length may be between 20 mm and 60 mm, or more specifically between 35 mm and 45 mm. In a preferred embodiment, the fibre length is around 40 mm.

Thereby, the fibres are suitable for hydroentanglement and are of a length wherein wicking is optimised. In case the wicking is experienced along the surface of the fibre, or within the fibre itself, it is desirable to have a long fibre since each discontinuation results in decreased wicking. However, the fibre cannot be too long either since this would result in poorer bonding properties in the hydroentanglement process and/or strength properties.

A method of making a functional core cover according to the invention may involve at least the steps of selecting a fraction of lyocell fibres, preparing a fibrous web of said selected fibres, and moving said fibrous web into a hydroentanglement section, whereby the fibres become mutually bonded in a non-woven fabric, and a majority of the fibres become oriented in the same direction as a result of the selected fraction of fibres, the preparation of the fibrous web, and the movement of the fibrous web into the hydroentanglement section.

The selection of fractions may be made according to the previously disclosed ranges, or more specifically according to the previously disclosed preferred embodiments. The subsequent step of bonding the selected fibres in a hydroentanglement process involves disposing the fibres in a fibrous web on a conveyor belt followed by the introduction of high-pressure water jets penetrating said fibrous web, the water jets entangling and thereby bonding the fibres into a non-woven fabric, or core cover, according to the invention. The use of carding machines and/or disposition of the fibres on a moving conveyor belt causes the fibres to be oriented primarily in what is known as the machine direction (i.e. the fibres are oriented in the same direction), which is the travelling direction of the conveyor belt. Conversely, the direction perpendicular to the machine direction is known as the cross direction. The tensile strength of the fabric in the machine direction (or the direction in which a majority of the fibres are oriented) is higher than the tensile strength of the fabric in the cross direction due to the frictional forces between the fibres.

A diaper comprising an absorbent core, characterised in that said absorbent core is covered by a functional core cover according to the invention.

By a diaper should be understood an absorbent article designed for wear by a human being. The diaper may for example be worn by not yet toilet-trained babies or by people suffering from incontinence. The absorbent core is the core of the diaper, i.e. the part of the diaper doing the actual absorption which may be considered the main purpose of a diaper.

Thereby, the diaper benefits from the wicking and strength advantages of the functional core cover as described previously.

### SHORT LIST OF THE DRAWINGS

In the following, example embodiments are described according to the invention, where
Fig. 1 illustrates a diaper suitable for being used in combination with a functional core cover according to the invention,
Fig. 2 illustrates the process of liquid wicking in a functional core cover according to the invention.
Fig. 3 illustrates a possible fabrication method for making a functional core cover according to the invention.
Fig. 4 illustrates the concept of wicking in prior art and in a functional core cover according to the invention.

### DETAILED DESCRIPTION OF DRAWINGS

In the following, the invention is described in detail through embodiments thereof that should not be thought of as limiting to the scope of the invention.

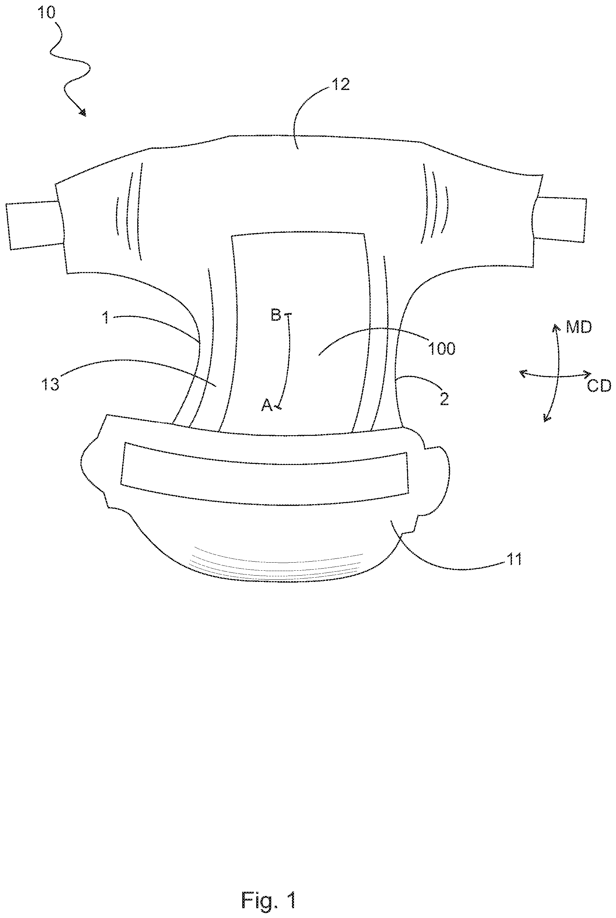

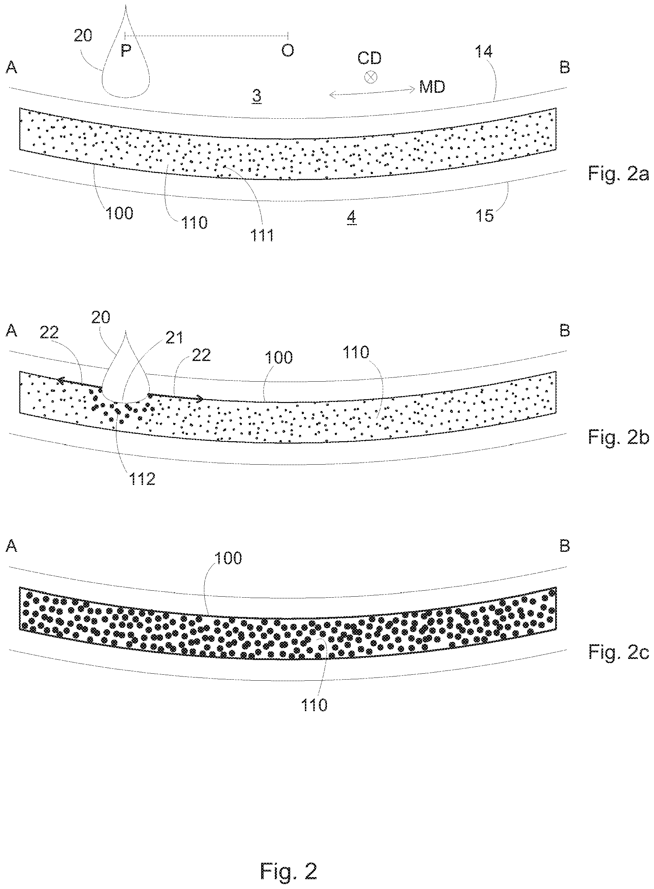

Fig. 1 illustrates a diaper 10 suitable for use in combination with a functional core cover 100 according to the invention. It should be noted that the figure is included for illustrative purposes only and does not explicitly disclose features of the functional core cover 100 as such. Further, it should be noted that the use of the functional core cover 100 is not limited to diapers only, but that said functional core cover 100 may be used in other products as well, for example in hygiene towels, bandages, or wipes. However, in a diaper as illustrated, the machine direction MD of the functional core cover 100 is preferably oriented, such that it extends from a front side 11 to a reverse side 12 of the diaper 10. Consequently, the cross direction CD extends from a first leg opening 1 to a second leg opening 2. In the diaper 10 as illustrated, the leg openings 1,2 are open since the diaper 10 is open, i.e. the ears of the reverse side 12 are not fastened on the front side 11. The machine direction MD and the cross direction CD are included for reference, but refer to the functional core cover 100 only since other parts of the diaper 100 may comprise fabric of different types. The line A-B indicates a series of side views discussed in relation to Fig. 2.

The functional core cover 100 encapsulates an absorbent core (not shown explicitly) suitable for absorbing expelled liquid (not shown). Preferably, the absorbent core is arranged in a bottom part 13 of the diaper 10, the bottom part 13 extending between the front side 11 and the reverse side 12. Preferably, the absorbent core is substantially flat and flexible, such that it may adjust to the shape of the diaper and the movements and contours of the wearer. The functional core cover 100 may be arranged below a cushioning layer (not shown). The cushioning layer may be omitted by the functional core cover 100 in certain embodiments of the invention, whereby the diaper may be made lighter and more flexible. The functional core cover 100 may in itself function as a cushioning layer thus having a dual function.

Fig. 2 illustrates a series of side views of a diaper taken along the line A-B in Fig. 1. References to A and B are included in Fig. 2 for illustrating the orientation of the side view. Despite again referring to a diaper, the skilled person notes that the invention relates to the functional core cover as such. In other words, the skilled person acknowledges that a functional core cover according to the invention may be utilised in other absorbent products, especially products used in the health care industry or in hygiene.

The series of side views illustrate the process of liquid absorption, and how a functional core cover according to the invention improves the utilisation of the absorbent core. The side views are taken from a diaper comprising a cushioning layer 14, but as it was mentioned above, the functional core cover itself may provide sufficient cushioning thereby removing the need for further layers. Preferably, the cushioning layer 14 is oriented adjacent the skin 3 of the wearer. The functional core cover 100 is shown as a box covering/encapsulating the absorbent core 110. The absorbent core 110 is illustrated using a plurality of dots 111 for highlighting a preferred embodiment wherein the absorbent core 110 comprises granules of SAP. It should be noted that the absorbent core 110 may further comprise cellulosic fluff pulp (not shown) functioning as a carrier for the SAP and for providing further cushioning and absorbency. A droplet 20 is included for illustrating how liquid is wicked and absorbed by the functional core cover 100 and the absorbent core 110 in combination. A back sheet 15 shields the diaper from the environment 4.

In Fig. 2a, a droplet 20 is expelled onto the diaper. The displaced position P relative to an origin O of the absorbent core 110 illustrates a common situation in a diaper, wherein liquid is expelled either in the front or in the back by human physiology. Thus, this helps illustrating the obstacles in prior art.

In Fig. 2b, the droplet 20 has been partly absorbed by the absorbent core 110. This is illustrated by enlarged dots (saturated SAP) 112 in the vicinity of the droplet 20. This resembles the situation and problems in prior art: the absorption occurs in the vicinity of the liquid expelling (the droplet 20), whereas a large portion of the absorbent core 110 remains dry. It is an aspect of the invention to provide means for wicking liquid towards the dry portions of the absorbent core 110 in order to utilise the full potential of said absorbent core 110, and especially the SAP.

According to the present invention, the situation shown in Fig. 2b merely represents an intermediate step, wherein the preliminary absorption occurs. During this intermediate step, liquid is partly absorbed by the absorbent core 110, especially by the SAP, whereas a remaining part of the liquid is absorbed by the functional core cover 100. Thereby, the liquid is absorbed by any kind of medium, rather than overflowing the absorbent core 110, i.e. no liquid remains unabsorbed until SAP of the absorbent core 110 becomes active. Concurrently, the liquid absorbed by the functional core cover 100 is wicked away from the point of depletion 21, i.e. the local area where liquid is expelled. The wicking 22 is illustrated by a set of arrows pointing in opposite directions away from the point of depletion 21.

In Fig. 2c, the liquid wicked by the functional core cover 100 has been wicked towards all parts of the absorbent core 110. The skilled person would understand that this is a gradual process, where the absorbent core/SAP gradually absorbs liquid until it has been saturated. When saturated, liquid is wicked further away towards unsaturated SAP, and so on. Further, the skilled person would understand that the liquid initially absorbed by the functional core cover 100 is eventually absorbed by the absorbent core 110, such that said absorbent core 110 holds the majority of the liquid, but that the functional core cover 100 functions as an intermediate absorption medium. As illustrated by Fig. 2c, the absorbent core 110 has absorbed the entire droplet, despite said droplet being arranged in a displaced position P relative to an origin O of the absorbent core 110. In prior art, this would not be the case following a single expelling of liquid.

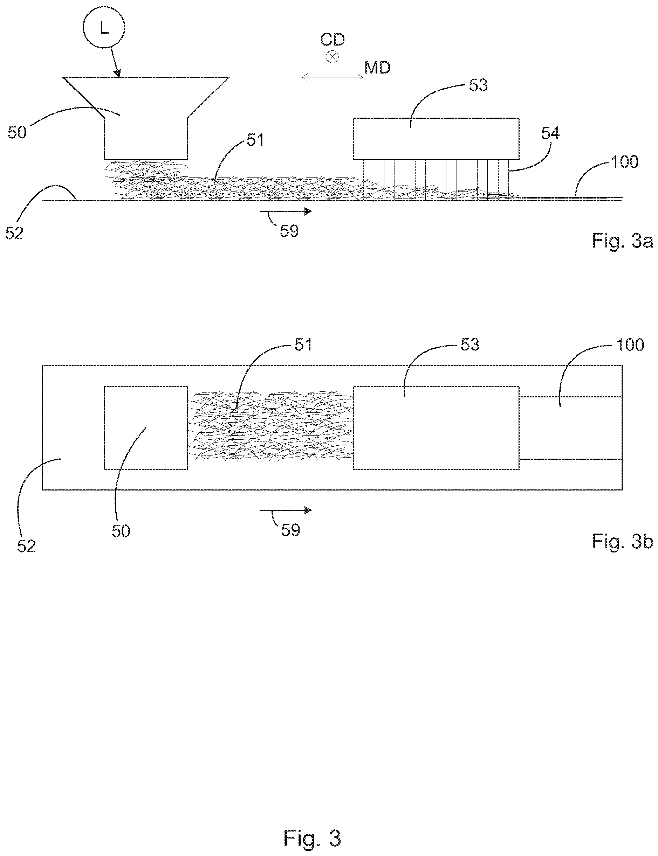

Fig. 3 illustrates the process of making a functional core cover 100 according to the invention. Fig. 3a illustrates a side view of the process, whereas Fig. 3b illustrates a top view of the embodiment shown in Fig. 3a.

At least one carding machine 50 is provided with the selected fibres, i.e. at least a fraction comprising lyocell fibres L. The carding machine 50 lays a fibrous web 51 comprising the fibres according to the invention on a conveyor belt 52, said conveyor belt 52 moving the fibrous web 51 into a hydroentanglement section 53. The direction of the conveyor belt 52 is indicated by the arrow 59. The hydroentanglement section 53 applies a plurality of water jets 54 onto the fibrous web 51, whereby the fibres within said fibrous web 51 are bonded/entangled. The end product of the process is a non-woven functional core cover 100 according to the invention which is bonded using hydroentanglement.

It should be understood, that the above-disclosed process of making a functional core cover according to the invention is not limiting to the scope of the invention.

When making the functional core cover 100 according to the above-described process, a majority of the fibres are oriented primarily in the machine direction MD, i.e. the direction identical to the orientation of the conveyor belt 52 carrying the fibrous web 51 into the hydroentanglement section 53 and the direction in which the fibrous web flows into the hydroentanglement section 53. This resulting orientation of the fibres in the functional core cover 100 may be utilised in the implementation of said functional core cover 100 in a diaper as described above. More precisely, if the functional core cover 100 is oriented in the diaper such that the machine direction MD is parallel to the direction extending from a front side to a reverse side of said diaper, wicking is enhanced, since liquid wicks along the length of fibres more easily than across fibres. The combination of fibre densities and lengths may further ensure that a majority of the fibres become oriented in the same direction. The corresponding cross direction CD being perpendicular to the machine direction MD is included for reference.

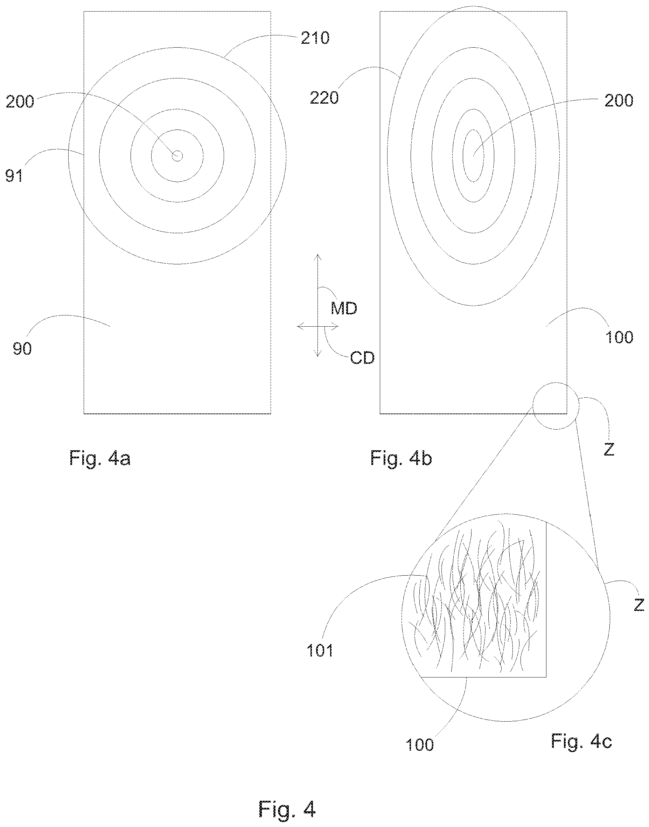

Fig. 4 illustrates the concept of wicking in a prior art core cover 90 (Fig. 4a) compared to wicking in a functional core cover 100 (Fig. 4b) according to the invention from a top view perspective. The machine direction MD and the cross direction CD have been included for reference. The rectangle illustrates a common shape of a prior art core cover 90 and a functional core cover 100 when used in an absorbent product, e.g. a diaper, where the shape ensures a broad coverage of the body. A liquid depletion 200 is shown to have its centre displaced from the centre of the rectangle, such that the need for enhanced wicking properties is highlighted. In prior art 90 (Fig. 4a), the liquid depletion is seen to form concentric circles 210, where the increase of the radii of the circles illustrates how the liquid is distributed in time. After a while, part of the liquid will reach the edge 91 of the rectangle (core cover) which illustrates a leakage situation. However, once said part of the liquid has reached the edge 91, a large part of the rectangle (i.e. core cover) remains dry - especially the bottom half of the illustration in Fig. 4a. On the other hand, in a functional core cover 100 (Fig. 4b) according to the invention, the wicking properties ensure that the liquid has a tendency to wick in the machine direction MD, i.e. the direction in which the fibres are oriented in the functional core cover 100. This is illustrated by the concentric ellipses 220. This reduces the risk of leakage while at the same time ensures that the liquid is distributed further away from the point of depletion. When distributed further away, more SAP may absorb the liquid, i.e. the functional core cover 100 provides a way to utilise the SAP present in the absorbent core (not shown) encapsulated/covered by the functional core cover to a greater extent than known from prior art.

Fig. 4c illustrates a zoom Z of a part of the functional core cover 100 according to the invention. In the zoom Z, part of the fibres 101 constituting the functional core cover 100 are shown. The orientation of the fibres 101 is particularly illustrated: although being flexible, i.e. bending in the illustration, the main orientation of the fibres is identical to a majority of the fibres. This common orientation is preferably aligned with the machine direction MD of the fabric, but may in general be any direction and may thus for example be independent from the manufacturing technique. The orientation may be expressed in terms of the tensile strength of the fabric, where said tensile strength is greater in the machine direction MD (or any direction in which a majority of the fibres are oriented) than in the cross direction CD (the direction perpendicular to the direction in which a majority of the fibres are oriented).

### REFERENCE NUMBERS

- A: Point A in cut A-B
- B: Point B in cut A-B
- CD: Cross Direction
- L: Lyocell fibres
- MD: Machine Direction
- O: Origin
- P: Displaced position
- Z: Zoom
- 1: First leg opening
- 2: Second leg opening
- 3: Skin
- 4: Environment
- 10: Diaper
- 11: Front side
- 12: Reverse side
- 13: Bottom part
- 14: Cushioning layer
- 15: Back sheet
- 20: Droplet
- 21: Point of depletion
- 22: Wicking direction
- 50: Card
- 51: Fibrous web
- 52: Conveyor belt
- 53: Hydroentanglement section
- 54: Water jets
- 59: Moving direction of conveyor belt 52
- 90: Prior art core cover
- 91: Edge of prior art core cover 90
- 100: Functional core cover
- 101: Fibres of functional core cover 100
- 110: Absorbent core
- 111: Super Absorbent Polymer (SAP)
- 112: Saturated SAP
- 200: Liquid depletion
- 210: Concentric circles
- 220: Concentric ellipses

## Claims

1. An absorbent article comprising a non-woven functional core cover encapsulating an absorbent core, said functional core cover comprising at least a first fraction comprising lyocell fibres, where a majority of the fibres constituting the functional core cover are oriented in the same direction, **characterised in that** the fibres in said functional core cover are mutually bonded via entanglement.

2. An absorbent article according to claim 1, wherein said functional core cover further comprises a second fraction comprising viscose rayon fibres.

3. An absorbent article according to any of the preceding claims, wherein the non-woven core cover is made in a hydroentanglement process.

4. An absorbent article according to any of the preceding claims, wherein a majority of the fibres of said functional core cover are oriented in the machine direction of the functional core cover, the machine direction being the direction in which a precursor to the functional core cover flows into a hydroentanglement section for bonding in the hydroentanglement process.

5. An absorbent article according to any of the preceding claims, wherein said functional core cover is flat, patterned, or apertured.

6. An absorbent article according to any of the preceding claims, wherein said functional core cover further comprises a third fraction comprising polypropylene fibres and/or polyester fibres.

7. An absorbent article according to any of the preceding claims, wherein the basis weight of said functional core cover is between 15 gsm and 30 gsm.

8. An absorbent article according to any of the preceding claims, wherein the density of the fibres of the non-woven functional core cover is between 1 dtex and 3.3 dtex.

9. An absorbent article according to any of the preceding claims, wherein the fibre length in the non-woven functional core cover is between 20 mm and 60 mm, or more specifically between 35 mm and 45 mm.

10. A method of making an absorbent article according to any of the preceding claims, **characterised in that** the method comprises at least the steps of
- making a functional core cover by:
- selecting a fraction of lyocell fibres,
- preparing a fibrous web of said selected fibres, and
- moving said fibrous web into a hydroentanglement section, whereby the fibres become mutually bonded in a non-woven fabric, and a majority of the fibres become oriented in the same direction, and
- encapsulating an absorbent core with said functional core cover.

11. An absorbent article according to any of the claims 1-9, wherein the absorbent article is a diaper encapsulating an absorbent core covered by a functional core cover.

## Patentansprüche

1. Ein absorbierender Artikel, der eine ungewebte funktionelle Kernabdeckung umfasst, die einen absorbierenden Kern einkapselt, wobei jene funktionelle Kernabdeckung mindestens eine erste Fraktion umfasst, die Lyocell-Fasern umfasst, wo eine Mehrheit der Fasern, welche die funktionelle Kernabdeckung bilden, in die gleiche Richtung orientiert sind, **dadurch gekennzeichnet, dass** die Fasern in jener funktionellen Kernabdeckung durch Verflechtung miteinander verbunden sind.

2. Ein absorbierender Artikel gemäß Anspruch 1, wobei jene funktionelle Kernabdeckung ferner eine zweite Fraktion umfasst, die Viskose-Rayon-Fasern umfasst.

3. Ein absorbierender Artikel gemäß einem der vorhergehenden Ansprüche, wobei die ungewebte Kernabdeckung in einem Hydroverflechtungsverfahren hergestellt wird.

4. Ein absorbierender Artikel gemäß einem der vorhergehenden Ansprüche, wobei ein Großteil der Fasern jener funktionellen Kernabdeckung in der Maschinenrichtung der funktionellen Kernabdeckung ausgerichtet sind, wobei die Maschinenrichtung die Richtung ist, in der ein Vorläufer der funktionellen Kernabdeckung in einen Hydroverflechtungungsabschnitt zur Verbindung im Hydroverflechtungsverfahren fließt.

5. Ein absorbierender Artikel gemäß einem der vorhergehenden Ansprüche, wobei jene funktionelle Kernabdeckung flach, strukturiert oder mit Öffnungen versehen ist.

6. Ein absorbierender Artikel gemäß einem der vorhergehenden Ansprüche, wobei jene funktionelle Kernabdeckung ferner eine dritte Fraktion umfasst, die Polypropylenfasern und/oder Polyesterfasern umfasst.

7. Ein absorbierender Artikel gemäß einem der vorhergehenden Ansprüche, wobei das Basisgewicht jener funktionellen Kernabdeckung zwischen 15 g/m² und 30 g/m² liegt.

8. Ein absorbierender Artikel gemäß einem der vorhergehenden Ansprüche, wobei die Dichte der Fasern der ungewebten funktionellen Kernabdeckung zwischen 1 dtex und 3,3 dtex liegt.

9. Ein absorbierender Artikel gemäß einem der vorhergehenden Ansprüche, wobei die Faserlänge in der ungewebten funktionellen Kernabdeckung zwischen 20 mm und 60 mm beträgt, oder insbesondere zwischen 35 mm und 45 mm beträgt.

10. Ein Verfahren zur Herstellung eines absorbierenden Artikels gemäß einem der vorhergehenden Ansprüche, **dadurch gekennzeichnet, dass** das Verfahren mindestens die Schritte von:
- Herstellen einer funktionellen Kernabdeckung durch:
- Auswählen einer Fraktion von Lyocell-Fasern,
- Herstellen eines Faservlieses aus jenen ausgewählten Fasern, und
- Bewegen jenes Faservlieses in einen Hydroverflechtungsabschnitt, wodurch die Fasern gegenseitig zu einem ungewebten Stoff verbunden werden und ein Großteil der Fasern in dieselbe Richtung ausgerichtet werden, und
- Einkapseln eines absorbierenden Kerns mit jener funktionellen Kernabdeckung umfasst.

11. Ein absorbierender Artikel gemäß einem der Ansprüche 1-9, wobei der absorbierende Artikel eine Windel ist, die einen absorbierenden Kern einkapselt, der von einer funktionellen Kernabdeckung bedeckt ist.

## Revendications

1. Article absorbant comprenant une couverture d'âme fonctionnelle non-tissée encapsulant un coeur absorbant, ladite couverture d'âme fonctionnelle comprenant au moins une première fraction de fibres de lyocell, où une majorité des fibres constituant la couverture d'âme fonctionnelle sont orientées dans la même direction, **caractérisé en ce que** les fibres dans ladite couverture d'âme fonctionnelle sont mutuellement liées par enchevêtrement.

2. Article absorbant selon la revendication 1, dans lequel ladite couverture d'âme fonctionnelle comprend en outre une deuxième fraction comprenant des fibres de rayonne viscose.

3. Article absorbant selon l'une quelconque des revendications précédentes, dans lequel la couverture d'âme non-tissée est fabriquée dans un processus d'hydroenchevêtrement.

4. Article absorbant selon l'une quelconque des revendications précédentes, dans lequel une majorité des fibres de ladite couverture d'âme fonctionnelle sont orientées dans le sens machine de la couverture d'âme fonctionnelle, le sens machine étant le sens dans lequel un précurseur de la couverture d'âme fonctionnelle passe dans une section d'hydro-enchevêtrement pour une liaison dans le processus d'hydroenchevêtrement.

5. Article absorbant selon l'une quelconque des revendications précédentes, dans lequel ladite couverture d'âme fonctionnelle est plate, à motifs ou ajourée.

6. Article absorbant selon l'une quelconque des revendications précédentes, dans lequel ladite couverture d'âme fonctionnelle comprend en outre une troisième fraction comprenant des fibres de polypropylène et/ou des fibres de polyester.

7. Article absorbant selon l'une quelconque des revendications précédentes, dans lequel le grammage de ladite couverture d'âme fonctionnelle est compris entre 15 g/m² et 30 g/m².

8. Article absorbant selon l'une quelconque des revendications précédentes, dans lequel la densité des fibres de la couverture d'âme fonctionnelle non-tissée est comprise entre 1 dtex et 3,3 dtex.

9. Article absorbant selon l'une quelconque des revendications précédentes, dans lequel la longueur des fibres dans la couverture d'âme fonctionnelle non-tissée est comprise entre 20 mm et 60 mm, ou plus spécifiquement entre 35 mm et 45 mm.

10. Procédé de fabrication d'un article absorbant selon l'une quelconque des revendications précédentes, **caractérisé en ce que** le procédé comprend au moins les étapes consistant à
- fabriquer une couverture d'âme fonctionnelle par
- sélection d'une fraction de fibres de lyocell,
- préparer une nappe fibreuse desdites fibres sélectionnées, et
- déplacer ladite nappe fibreuse dans une section d'hydro-enchevêtrement, grâce à quoi les fibres deviennent mutuellement liées dans un tissu non-tissé, et une majorité des fibres s'orientent dans la même direction, et
- encapsuler un coeur absorbant avec ladite couverture d'âme fonctionnelle.

11. Article absorbant selon l'une quelconque des revendications 1 à 9, dans lequel l'article absorbant est une couche encapsulant un coeur absorbant recouvert par une couverture d'âme fonctionnelle.
